# EUROPÄISCHE PATENTSCHRIFT

(11) **EP 0 344 418 B1**
(45) Veröffentlichungstag und Bekanntmachung des Hinweises auf die Patenterteilung: **26.05.1993**
(21) Anmeldenummer: 89104881.1
(22) Anmeldetag: 18.03.1989
(51) Int. Cl.: C07C 67/00, C07C 69/33, C07C 69/58, C11C 3/00

(54) **Verfahren zur Herstellung von nichtionogenen Tensiden**
Process for the preparation of non-ionogenic tensides
Procédé de préparation de dérivés tensio-actifs non ionogènes

(30) Priorität: 30.05.1988 DE 3818293
(43) Veröffentlichungstag der Anmeldung: 06.12.1989
(73) Patentinhaber: DEUTSCHE SOLVAY-WERKE GMBH, 42697 Solingen (DE)
(72) Erfinder: Jakobson, Gerald, Dr. Dipl.-Chem., D-4134 Rheinberg 2 (DE); Siemanowski, Werner, Dr. Dipl.-Chem., D-4134 Rheinberg 1 (DE); Uhlig, Karl-Heinz, D-4150 Krefeld-Traar (DE)
(74) Vertreter: Seiler, Siegfried

(56) Entgegenhaltungen:
- EP-A- 0 070 535
- DE-B- 1 808 229
- US-A- 3 936 391
- US-A- 4 035 513
- SOVIET INVENTIONS ILLUSTRATED, Sektion Ch, Woche D18, 10. Juni 1981; DERWENT PUBLICATIONS LTD., London, D25
- CHEMICAL ABSTRACTS, Band 41, Nr. 8, 20. April 1947, Columbus, Ohio, USA; M.ISTIN, "The esters of polyglyceric alcohols and fatty acids", Spalten 2392,2393,2394

## Beschreibung

Die vorliegende Erfindung betrifft ein Verfahren zur Herstellung von nichtionogenen Tensiden aus Fettsäurealkylestern, Mono- oder Polyhydroxyfettsäurealkylester mit C₆ - C₂₂ in der Fettsäurekomponente und C₁ - C₄ in der Esterkomponente, die im alkalischen Medium mit einem oder mehreren Isopropylidenderivaten eines Polyglycerins umgesetzt werden, wobei bestimmte Verfahrensbedingungen zur Herstellung der Fettsäureester des Polyglycerins einzuhalten sind.

Es ist bekannt, daß gemäß US-A-4 035 513 Fettsäureestergemische hergestellt werden können, die immer endständige Fettsäuregruppen aufweisen. Diese endständigen Fettsäureester werden dadurch gebildet, daß die Umsetzung der Polyhydroxyalkohole unmittelbar mit den Fettsäuren erfolgt, ohne daß vorher Schutzgruppen angelagert werden. Dabei wird keine gezielte Anlagerung von Schutzgruppen durchgeführt, so daß reine vicinale Ester des Diglycerins mittelständige Monoester des Triglycerins oder mittelständige Mono- oder Diester des Tetraglycerins nicht erhalten werden.

Es ist weiterhin bereits bekannt, Fettsäureester der Polyglycerine durch mehrtägiges Erhitzen von Diglycerin mit einem großen Überschuß von Fettsäuren, z. B. als Tetraester von Laurin-, Palmi-tin-, Stearin- und ölsäure in Form brauner, fester oder öliger Verbindungen herzustellen ((vgl. C.A. 41,2392 (1947)). Infolge der langen Erhitzungsdauer können nur stark verunreinigte Produkte mit sehr schlechter Ausbeute erhalten werden. Die so hergestellten Tetraester sind nicht als Tenside verwendbar. Man hat daher versucht, entsprechende Verbindungen aus Isopropylidendiglycerin mit Stearylchlorid in Chloroform zu erhalten. Jedoch ist der dabei erforderliche Arbeitsaufwand beträchtlich, da nach der Umsetzung das Gemisch zu einem Brei erstarrt, das nach 36 Std. mit Benzol aufgenommen werden muß. Nach Abtrennung der wäßrigen Lösung und Waschen mit Wasser, die überschüssige Stearinsäure durch Schütteln mit 10%iger Natriumbicarbonatlösung entfernt, über Natriumsulfat getrocknet und das Lösungsmittel abdestilliert werden muß. Der dabei entstehende Rückstand muß zwei- oder mehrfach mit Alkoholen umkristallisiert werden. Nachteilig bei diesem Verfahren ist weiterhin, daß Hydrochloride in molaren Verhältnissen anfallen, die entweder weiter verarbeitet werden müssen oder umweltbelastende Stoffe darstellen.

Ziel und Aufgabe der vorliegenden Erfindung war es daher, ein verbessertes Verfahren zur Herstellung nichtionogener Tenside, insbesondere Fettsäureester der Polyglycerine, aus Fettsäurealkylestern, Mono- oder Polyhydroxyfettsäurealkylestern mit C₆ bis C₂₂ in der Fettsäurekomponente und C₁ bis C₄ in der Esterkomponente durch Umsetzung mit einem oder mehreren Isopropylidenderivaten eines Polyglycerins zu erhalten. Insbesondere sollte ein Verfahren gefunden werden, das gezielte großtechnische Veresterungen ermöglicht. Weiterhin sollten evtl. im Überschuß eingesetzte Reaktionsprodukte oder -komponenten zu einem Teil in das Verfahren rückführbar sein.

Erfindungsgemäß wurde festgestellt, daß diesen Zielen und Aufgaben ein Verfahren zur Herstellung nichtionogener Tenside gerecht wird, wobei die entsprechenden Polyglycerinmono- und/oder -polyfettsäureester aus Fettsäurealkylestern (einschließlich Mono- oder Polyhydroxyfettsäurealkylestern) mit C₆ - C₂₂ in der Fettsäurekomponente und C₁ - C₄ in der Esterkomponente erhalten werden, die im alkalischen Medium mit einem oder mehreren Isopropylidenderivaten eines Polyglycerins umgesetzt werden. Gemäß der Erfindung wird die Umsetzung bei Temperaturen von 140 - 220 °C, vorzugsweise 170 - 200 °C, und im Vakuum bei 950 - 5 mbar, vorzugsweise bei 500 - 10 mbar, durchgeführt, wobei der entstehende C₁- C₄ Alkohol durch Destillation entfernt, vorzugsweise kontinuierlich entfernt und das nachfolgend gereinigte, vorzugsweise durch Filtration, Zentrifugation, Destillation und/oder fraktionierte Destialltion gereinigte Reaktionsprodukt hydrolisiert wird, indem mindestens eine Isopropylidengruppe des Reaktionsproduktes bei 20 bis 100°C, vorzugsweise 50 bis 80°C, sowie bei Normal-, Unter-oder Überdruck durch saure Hydrolyse gespalten wird.

Wird die Reaktion bei unter 140 °C durchgeführt, so treten keine ausreichenden Reaktionsgeschwindigkeiten auf, während bei Temperaturen von über 220 °C ein hoher Prozentsatz unerwünschter Nebenprodukte anfällt. Führt man andererseits die Reaktion bei Normaldruck durch, tritt eine längere Umsetzungsdauer auf und ebenfalls ein höherer Prozentsatz unerwünschter Nebenprodukte. Als Fettsäurealkylester werden gesättigte oder ungesättigte, verzweigte oder unverzweigte Fettsäurealkylester eingesetzt, u.a. Ester von Vorlauffettsäuren C₆-C₁₀, Laurinsäure, Myristinsäure, Cocosfettsäure, Stearinsäure, Behensäure und/oder 2- Ethylhexansäure, Isostearinsäure, Palmölfettsäure, Ölfettsäure, Sojaölfettsäure und/oder Linolsäure.

Als Hydroxyfettsäurealkylester werden u.a. 12-Hydroxystearinsäureäthylester und/oder Rizinolfettsäureester eingesetzt.

Als alkalisch reagierende Verbindung zur Beschleunigung der Reaktion wird bevorzugt mindestens ein Alkali- und/oder Erdalkalihydroxid, -bicarbonat, und/oder -carbonat und/oder -alkoholat und/oder eine Alkali- und/oder Erdalkaliseife zugefügt.

Nach einer bevorzugten Ausführungsform wird die Umsetzung der Fettsäurealkylester, Mono- oder Polyhydroxyfettsäurealkylester mit einem oder mehreren Isopropylidenderivaten eines Polyglycerins in Gegenwart von weniger als 5 Gew.-% Wasser, vorzugsweise weniger als 1 Gew.-% Wasser (bezogen auf die Gesamtmenge der umzusetzenden Verbindungen) durchgeführt. Gemäß dieser Ausführungsform können gewisse Nebenreaktionen vermindert werden, so daß der Gehalt an evtl. Verunreinigungen reduziert werden kann.

Als Isopropylidenderivate des Polyglycerins werden bevorzugt Mono- und/oder Diisopropylidenderivate des Di-, Tri- und/oder Tetraglycerins (ausgenommen Diisopropylidendiglycerin) eingesetzt.

Nach einer bevorzugten Ausführungsform des erfindungsgemäßen Verfahrens erfolgt die Umsetzung des Fettsäurealkylesters, Mono- und/oder Polyhydroxyfettsäurealkylesters mit einem Mono- und/oder Diisopropylidenderivat eines Di- (ausgenommen Diisopropylidendiglycerin), Tri- und/oder Tetraglycerins pro umzusetzende Hydroxylgruppe (bezogen auf die Mono- oder Diisopropylidenderivate des jeweiligen Polyglycerins) in einem 1,2 bis 3-fachen, vorzugsweise 1,5- bis 2,5-fachen, molaren Überschuß an Fettsäurealkylester. Durch diesen Überschuß gelingt es eine höhere Ausbeute zu erhalten.

Nach einer bevorzugten Ausführungsform wird im Falle der Umsetzung eines Mono- und/oder Diisopropylidenderivates des Di- und/oder Tetraglycerins mit Fettsäurealkylestern, Mono- oder Polyhydroxyfettsäurealkylstern ein 2,1 - 10-facher, vorzugsweise 4- bis 8-facher, molarer Überschuß an Mono-isopropylidendiglycerin und/oder Diisopropylidentetraglycerin (bezogen auf Fettsäurealkylester, Mono- oder Polyhydroxyfettsäurealkylester) eingesetzt. Dadurch wird der Gehalt an gebildeten Mono-estern stark erhöht.

Das Reaktionsprodukt wird bevorzugt nach der Umesterung durch Filtration bei Temperaturen von 20 bis 100°C, vorzugsweise 40 bis 70 °C, von nichtlöslichen Bestandteilen getrennt.

Nach einer bevorzugten Ausführungsform wird nach der Umsetzung des Fettsäurealkylesters, Mono-und/oder Polyhydroxyfettsäurealkylesters mit einem oder mehreren Iso- bzw. Diisopropylidenderivaten des Di-, Tri- und/oder Tetraglycerins (ausgenommen Diisopropylidendiglycerin) der Überschuß des nicht umgesetzten Fettsäurealkylesters, Mono- und/oder Polyhydroxyfettsäurealkylesters und/oder der nichtumgesetzten Iso- bzw. Diisopropylidenderivate des Di-, Tri- und/oder Tetraglycerins im Vakuum abdestilliert, vorzugsweise kontinuierlich abdestilliert und das zurückbleibende Umsetzungsprodukt in einem niedrigen Alkohol und/oder wasserhaltigem niedrigen Alkohol mit C₁-C₆, vorzugsweise mit C₂ - C₄, suspendiert und bei 20 bis 100 °C , vorzugsweise 50 bis 80 °C in Gegenwart eines sauren Katalysators hydrolysiert. Nach erfolgter Reaktion und Hydrolyse wird das sich bildende Lösemittelgemisch (Wasser, entstandenes Aceton, Alkohol) abdestilliert. Bevorzugt wird dabei als niedriger Alkohol n-Butanol verwendet.

Vor der Abdestillation des Lösemittels wird bevorzugt die im Reaktionsgemisch vorhandene Säure oder der saure Katalysator neutralisiert und/oder entfernt und/oder das Lösemittelgemisch abdestilliert.

Nach einer bevorzugten Ausführungsform erfolgt die Neutralisation durch Zugabe eines Anionenaustauschers (Anionenaustaschermasse oder -mittel) und der Anionenaustauscher wird nachfolgend abfiltriert.

### Ausführungsbeispiele

1.1 Umsetzung von Fettsäurealkylestern mit Diiso propylidentriglycerin
   1,52 kg (5 mol) Ölsäuremethylester und 25 g (0,2 mol) Kaliumcarbonat werden in einen 4 1-Kolben gegeben und unter Rühren auf ca. 160 °C aufgeheizt. Bei ca. 100 mbar Unterdruck werden evtl. vorhandene geringe Mengen an Wasser abdestilliert. Daraufhin werden 820 g (2,5 mol) Diisopropylidentriglycerin zugegeben und die Reaktionstemperatur auf 180 °C - 190 °C erhöht. Bei 400 - 50 mbar wird das entstehende Methanol destillativ entfernt. Nach 4 - 5 stündiger Reaktionszeit wird der Ansatz auf ca. 70 °C abgekühlt und ausgefallene Bestandteile abfiltriert.
   Überschüssiger Fettsäurealkylester sowie nicht umgesetztes Diisopropylidentriglycerin werden bei ≦ 0,4 mbar und ca. 160 °C Kopftemperatur destillativ entfernt. Das verbleibende Rohprodukt wird anschließend in einer Kurzwegverdampferanlage bei ≦ 0,1 mbar und 210°C Ölvorlauftemperatur feindestilliert.
1.2 Hydrolyse des Diisopropylidentriglycerinmonooleats zum Triglycerinmonooleat
   2,5 kg (ca. 4,4 mol) Diisopropylidentriglycerin-monooleat werden zu einer Mischung aus 250 ml Wasser und 2,0 l n-Butanol gegeben. Die Reaktionslösung wird mit 1 molarer Schwefelsäure auf ca. pH 3 eingestellt, auf 70 °C erwärmt und für 4 - 5 h gerührt. Nach Beendigung der Reaktion wird der Ansatz mit soviel OH⁻-beladenem Anionenaustauscher versetzt, bis die Reaktionslösung neutral ist.
   Der Ionenaustauscher wird abfiltriert, das Filtrat gegebenenfalls mit Aktivkohle gebleicht und die Leichtsieder (n-Butanol, Wasser, entstandenes Aceton) im Vakuum abdestilliert.
2.1 Umsetzung von Fettsäurealkylestern mit Monoisopropylidendiglycerin
   300 g (ca. 1 mol) Palmitinsäuremethylester und 0,5 g Lithiumhydroxid · 1 H₂O werden in einen 2 l-Kolben gegeben und unter Rühren auf 160 °C erwärmt. Bei 150 mbar wird das (aus dem Katalysator sowie mit dem Fettsäureester eingeschleppten) Wasser destillativ entfernt.
   Daraufhin werden 1,031 kg (5 mol) Monoisopropylidendiglycerin zugegeben und die Reaktionstemperatur auf 190 °C erhöht. Bei 400 - 50 mbar (geringer Druck zum Ende der Reaktion) wird das entstehende Methanol destillativ entfernt. Nach 3-stündiger Reaktionszeit wird der Ansatz auf 45 °C abgekühlt und ausgefallene Bestandteile abfiltriert.
   Überschüssiges Monoisopropylidendiglycerin wird bei ≦ 0,2 mbar und ca. 140 °C Ölvorlauftemperatur in einer Kurzwegverdampferanlage destillativ entfernt, das verbleibende Rohprodukt in der gleichen Anlage bei ≦ 0,2 mbar und 205°C Ölvorlauftemperatur abschließend feindestilliert.
2.2 Hydrolyse des Monoisopropylidendiglycerinmonopalmitats zum Diglycerinmonopalmitat
   1,5 kg Monoisopropylidendiglycerin-monopalmitat werden zu einer Mischung aus 200 ml Wasser und 2,0 l n-Butanol gegeben. Die Reaktionslösung wird mit 1 molarer Schwefelsäure auf ca. pH 3 eingestellt, auf 70 °C erwärmt und für 4 - 5 h gerührt. Nach Beendigung der Reaktion wird der Ansatz mit soviel OH⁻-beladenem Anionenaustauscher versetzt, bis die Reaktionslösung neutral ist.
   Der Ionenaustauscher wird abfiltriert, das Filtrat gegebenenfalls mit Aktivkohle gebleicht und die Leichtsieder (n-Butanol, Wasser, entstandenes Aceton) im Vakuum abdestilliert.

## Patentansprüche

1. Verfahren zur Herstellung von nichtionogenen Tensiden aus Fettsäurealkylestern, Mono- oder Polyhydroxyfettsäurealkylestern mit C₆ - C₂₂ in der Fettsäurekomponente und C₁ - C₄ in der Esterkomponente, die im alkalischen Medium mit einem oder mehreren Isopropylidenderivaten eines Polyglycerins umgesetzt werden, dadurch gekennzeichnet, daß die Umsetzung bei Temperaturen von
140 - 220 °C, vorzugsweise
170 - 200 °C,
und im Vakuum
bei 950 - 5 mbar, vorzugsweise
bei 500 - 10 mbar,
durchgeführt, der dabei entstehende C₁ - C₄ Alkohol durch Destillation entfernt, vorzugsweise kontinuierlich entfernt und das nachfolgend gereinigte, vorzugsweise durch Filtration, Zentrifugation, Destillation und/oder fraktionierte Destillation gereinigte Reaktionsprodukt hydrolisiert wird, indem mindestens eine Isopropylidengruppe des Reaktionsproduktes bei
20 bis 100°C, vorzugsweise
50 bis 80°C,
sowie bei Normal-, Unter- oder Überdruck durch saure Hydrolyse gespalten wird.

2. Verfahren nach Anspruch 1, dadurch gekennzeichnet, daß als alkalisch reagierende Verbindung zur Beschleunigung der Reaktion mindestens ein Alkali- und/oder Erdalkalihydroxid, -bicarbonat und/oder -carbonat und/oder -alkoholat und/oder eine Alkali- und/oder Erdalkaliseife zugefügt wird.

3. Verfahren nach Ansprüchen 1 und 2, dadurch gekennzeichnet, daß die Umsetzung der Fettsäurealkylester, Mono- oder Polyhydroxyfettsäurealkylester mit einem oder mehreren Isopropylidenderivaten eines Polyglycerins in Gegenwart von weniger als 5 Gew.-% Wasser, vorzugsweise weniger als 1 Gew.-% Wasser (bezogen auf die Gesamtmenge der umzusetzenden Verbindungen), durchgeführt wird.

4. Verfahren nach einem oder mehreren der Ansprüche 1 bis 3, dadurch gekennzeichnet, daß als Isopropylidenderivate des Polyglycerins Mono-und/oder Diisopropylidenderivate des Di-, Tri-und/oder Tetraglycerins (ausgenommen Diisopropylidendiglycerin) eingesetzt werden.

5. Verfahren nach einem oder mehreren der Ansprüche 1 bis 4, dadurch gekennzeichnet, daß die Umsetzung des Fettsäurealkylesters, Mono- oder Polyhydroryfettsäurealkylesters mit einem Mono-und/oder Diisopropylidenderivat eines Di-(ausgenommen Diisopropylidendiglycerin), Tri-und/oder Tetraglycerins pro umzusetzende Hydroxylgruppe (bezogen auf die Mono- oder Diisopropylidenderivate des jeweiligen Polyglycerins) in einem 1,2- bis 3-fachen, Vorzugsweise 1,5- bis 2,5-fachen molaren Überschuß an Fettsäurealkylester, Mono- oder Polyhydroxyfettsäurealkylester erfolgt.

6. Verfahren nach einem oder mehreren der Ansprüche 1 bis 5, dadurch gekennzeichnet, daß im Falle der Umsetzung eines Mono- und/oder Diisopropylidenderivates des Di-, Tri- und/oder Tetraglycerins mit Fettsäurealkylester ein 2,1 bis 10-facher, vorzugsweise 4- bis 8-facher, molarer Überschuß an Monoisopropylidendiglycerin und/oder Diisopropylidentriglycerin und/oder Diisopropylidentetraglycerin (bezogen auf Fettsäurealkylester, Mono- oder Polyhydroxyfettsäurealkylester) eingesetzt wird.

7. Verfahren nach einem oder mehreren der Ansprüche 1 bis 6, dadurch gekennzeichnet, daß das Reaktionsprodukt nach der Umesterung durch Filtration bei Temperaturen von
20 - 100 °C, vorzugsweise
40 - 70 °C,
von nichtlöslichen Bestandteilen getrennt wird.

8. Verfahren nach einem oder mehreren der Ansprüche 1 bis 7, dadurch gekennzeichnet, daß nach der Umsetzung des Fettsäurealkylesters, Mono-oder Polyhydroxyfettsäurealkylesters mit einem oder mehreren Isopropylidenderivaten des Di-, Tri- und/oder Tetraglycerins (ausgenommen Diisopropylidendiglycerin) der Überschuß des nicht umgesetzten Fettsäurealkylesters, Mono-oder Polyhydroxyfettsäurealkylesters und/oder des nicht umgesetzten Isopropylidenderivates des Diglycerins und/oder Diisopropylidenderivates des Tetraglycerins im Vakuum abdestilliert wird, vorzugsweise kontinuierlich abdestilliert wird, und das zurückbleibende Umsetzungsprodukt in einem niedrigen Alkohol und/oder wasserhaltigem niedrigen Alkohol
mit C₁ - C₆, vorzugsweise
mit C₂ - C₄,
suspendiert, bei 20 bis 100 °C, vorzugsweise 50 bis 80°C, in Gegenwart eines sauren Katalysators hydrolysiert und nach erfolgter Reaktion das Lösemittelgemisch (Wasser, entstandenes Aceton, Alkohol) abdestilliert wird.

9. Verfahren nach einem oder mehreren der Ansprüche 1 bis 8, dadurch gekennzeichnet, daß als niedriger Alkohol n-Butanol verwendet wird.

10. Verfahren nach einem oder mehreren der Ansprüche 1 bis 9, dadurch gekennzeichnet, daß vor der Abdestillation des Lösemittels die im Reaktionsgemisch vorhandene Säure oder der saure Katalysator neutralisiert und/oder entfernt und/oder das Lösemittelgemisch abdestilliert wird.

11. Verfahren nach einem oder mehreren der Ansprüche 1 bis 10, dadurch gekennzeichnet, daß die Neutralisation durch Zugabe eines Anionenaustauschers erfolgt und der Anionenaustauscher nachfolgend abfiltriert wird.

## Claims

1. A method for the preparation of non-ionogenic tensides from fatty acid alkyl esters, monohydroxy or polyhydroxy fatty acid alkyl esters with C₆ - C₂₂ in the fatty acid constituent and C₁ - C₄ in the ester constituent, which are reacted in alkaline medium with one or more isopropylidene derivatives of a polyglycerol, characterised in that the reaction is carried out at temperatures of
140 - 220°C, preferably
170 - 200°C,
and in a vacuum
at 950 - 5 mbar, preferably
at 500 - 10 mbar,
the resulting C₁ - C₄ alcohol is removed, preferably continuously removed, by distillation, and the reaction product subsequently purified, preferably purified by filtration, centrifugation, distillation and/or fractional distillation, is hydrolysed, by cleaving at least one isopropylidene group of the reaction product by acid hydrolysis at
20 to 100°C, preferably
50 to 80°C,
and at normal pressure, reduced pressure or excess pressure.

2. A method according to Claim 1, characterised in that at least one alkali and/or alkaline earth hydroxide, bicarbonate and/or carbonate and/or alcoholate and/or an alkali and/or alkaline earth soap is added as an alkaline-reacting compound for accelerating the reaction.

3. A method according to Claims 1 and 2, characterised in that the reaction of the fatty acid alkyl ester, monohydroxy or polyhydroxy fatty acid alkyl ester with one or more isopropylidene derivatives of a polyglycerol is carried out in the presence of less than 5% by weight water, preferably less than 1% by weight water (relative to the total quantity of the compounds to be reacted).

4. A method according to one or more of Claims 1 to 3, characterised in that monoisopropylidene and/or diisopropylidene derivatives of diglycerol, triglycerol and/or tetraglycerol (with the exception of diisopropylidene diglycerol) are used as isopropylidene derivatives of polyglycerol.

5. A method according to one or more of Claims 1 to 4, characterised in that the reaction of the fatty acid alkyl ester, monohydroxy or polyhydroxy fatty acid alkyl ester with a monoisopropylidene and/or diisopropylidene derivative of a diglycerol (with the exception of diisopropylidene diglycerol), triglycerol and/or tetraglycerol per hydroxyl group to be reacted (relative to the monoisopropylidene or diisopropylidene derivatives of the respective polyglycerol) takes place in a 1.2 to 3 times, preferably 1.5 to 2.5 times, molar excess of fatty acid alkyl ester, monohydroxy or polyhydroxy fatty acid alkyl ester.

6. A method according to one or more of Claims 1 to 5, characterised in that in the case of the reaction of a monoisopropylidene and/or diisopropylidene derivative of diglycerol, triglycerol and/or tetraglycerol with fatty acid alkyl ester a 2.1 to 10 times, preferably 4 to 8 times, molar excess of monoisopropylidene diglycerol and/or diisopropylidene triglycerol and/or diisopropylidene tetraglycerol (relative to fatty acid alkyl ester, monohydroxy or polyhydroxy fatty acid alkyl ester) is used.

7. A method according to one or more of Claims 1 to 6, characterised in that the reaction product after the transesterification is separated from insoluble constituents by filtration at temperatures of
20 - 100°C, preferably
40 - 70°C.

8. A method according to one or more of Claims 1 to 7, characterised in that after the reaction of the fatty acid alkyl ester, monohydroxy or polyhydroxy fatty acid alkyl ester with one or more isopropylidene derivatives of diglycerol, triglycerol and/or tetraglycerol (with the exception of diisopropylidene diglycerol) the excess of the non-reacted fatty acid alkyl ester, monohydroxy or polyhydroxy fatty acid alkyl ester and/or of the non-reacted isopropylidene derivative of diglycerol and/or diisopropylidene derivative of tetraglycerol is distilled off, preferably distilled off continuously, in a vacuum, and the remaining reaction product is suspended in a lower alcohol and/or water-containing lower alcohol
having C₁ to C₆, preferably
having C₂ to C₄,
is hydrolysed at 20 to 100°C, preferably 50 to 80°C, in the presence of an acid catalyst and the solvent mixture (water, resulting acetone, alcohol) is distilled off once the reaction has taken place.

9. A method according to one or more of Claims 1 to 8, characterised in that n-butanol is used as the lower alcohol.

10. A method according to one or more of Claims 1 to 9, characterised in that before the solvent has been distilled off the acid present in the reaction mixture or the acid catalyst is neutralised and/or removed and/or the solvent mixture is distilled off.

11. A method according to one or more of Claims 1 to 10, characterised in that the neutralisation takes place by addition of an anion exchanger and the anion exchanger is subsequently filtered off.

## Revendications

1. Procédé de préparation d'agents tensio-actifs non ionogènes à partir d'alkylesters d'acides gras, d'alkylesters d'acides gras mono ou polyhydroxylés en C₆-C₂₂ dans le composant acide gras et en C₁ à C₄ dans le composant ester par réaction avec un ou plusieurs dérivés d'isopropylidène d'une polyglycérine, caractérisé en ce que la réaction est réalisée à des températures de
140 - 220°C, de préférence
170 - 200°C,
et sous un vide de
950 - 5 mbars, de préférence
500 - 10 mbars,
l'alcool en C₁-C₄ correspondant est éliminé par distillation, de préférence éliminé en continu, et le produit réactionnel ensuite épuré, de préférence, par filtration, centrifugation, distillation et/ou distillation fractionnée est hydrolysé, tandis qu'un groupe isopropylidène du produit réactionnel est scindé par hydrolyse acide à
20 - 100°C, de préférence
50 - 80°C sous pression normale, dépression ou surpression.

2. Procédé selon la revendication 1, caractérisé en ce qu'on ajoute comme composé à réaction alcaline pour accélérer la réaction au moins un hydroxyde, un bicarbonate et/ou un carbonate et/ou un alcoolate de métal alcalin et/ou de métal alcalino-terreux et/ou un savon de métal alcalin et/ou de métal alcalino-terreux.

3. Procédé selon les revendications 1 et 2, caractérisé en ce que la réaction des alkylesters d'acides gras, des alkylesters d'acides gras mono ou polyhydroxylés avec un ou plusieurs dérivés d'isopropylidène d'une polyglycérine est effectuée en présence de moins de 5 % d'eau, de préférence moins de 1 % d'eau (par rapport à la quantité totale des composés participant à la réaction).

4. Procédé selon une ou plusieurs des revendications 1 à 3, caractérisé en ce qu'on utilise comme dérivés d'isopropylidène de polyglycérine des dérives de mono et/ou diisopropylidène de di, de tri et/ou de tétraglycérine (à l'exception de la diisopropylidènediglycérine).

5. Procédé selon une ou plusieurs des revendications 1 à 4, caractérisé en ce que la réaction de l'alkylester d'acide gras, de l'alkylester d'acide gras mono et/ou polyhydroxylé avec un dérivé de mono et/ou diisopropylidène d'une di (à l'exception de la diisopropylidènediglycérine), d'une tri et/ou d'une tétraglycérine par groupe hydroxyle réagissant (par rapport au dérivé de mono ou de diisopropylidène de la polyglycérine respective) se fait selon un excès molaire de 1,2 à 3 fois, de préférence 1,5 à 2,5 fois, d'alkylester d'acide gras, d'alkylester d'acide gras mono ou polyhydroxylé.

6. Procédé selon une ou plusieurs des revendications 1 à 5, caractérisé en ce qu'on utilise dans le cas de la réaction d'un dérivé de mono et/ou de diisopropylidène de la di, de la tri et/ou de la tétraglycérine avec des alkylesters d'acides gras, des alkylesters d'acides gras mono ou polyhydroxylés un excès molaire de 2,1 à 10 fois, de préférence 4 à 8 fois, de monoisopropylidènediglycérine et/ou de diisopropylidènetétraglycérine (par rapport aux alkylesters d'acides gras, aux alkylesters d'acides gras mono ou polyhydroxylés).

7. Procédé selon une ou plusieurs des revendications 1 à 6, caractérisé en ce que le produit réactionnel est de préférence après l'estérification séparé des composants non solubles par filtration à des températures de
20 à 100°C, de préférence
40 à 70°C.

8. Procédé selon une ou plusieurs des revendications 1 à 7, caractérisé en ce que, après la réaction de l'alkylester d'acide gras, de l'alkylester d'acide gras mono et/ou polyhydroxylé avec un ou plusieurs dérivés d'iso ou de diisopropylidène de la di, de la tri et/ou de la tétraglycérine (à l'exception de la diisopropylidènediglycérine), l'excès d'alkylester d'acide gras, d'alkylester d'acide gras mono et/ou polyhydroxylé n'ayant pas réagi et/ou de dérivés d'iso ou de diisopropylidène de la di, de la tri et/ou de la tétraglycérine non convertis est séparé par distillation sous vide, de préférence séparé par distillation en continu, et le produit réactionnel subsistant est mis en suspension dans un alcool inférieur et/ou dans un alcool inférieur aqueux
en C₁-C₆, de préférence
en C₂-C₄,
et hydrolysé à 20 - 100°C, de préférence 50 - 80°C, en présence d'un catalyseur acide et, après réaction couronnée de succès, le mélange de solvants qui s'est formé (eau, acétone produite, alcool) est séparé par distillation.

9. Procédé selon une ou plusieurs des revendications 1 à 8, caractérisé en ce qu'on utilise comme alcool inférieur du n-butanol.

10. Procédé selon une ou plusieurs des revendications 1 à 9, caractérisé en ce que, avant la séparation par distillation du solvant, l'acide présent dans le mélange réactionnel ou le catalyseur acide est de préférence neutralisé et/ou éliminé et/ou le mélange de solvants séparé par distillation.

11. Procédé selon une ou plusieurs des revendications 1 à 10, caractérisé en ce que la neutralisation se fait par addition d'un échangeur d'anions et l'échangeur d'anions est ensuite séparé par filtration.
